(19) Europäisches Patentamt
European Patent Office
Office européen des brevets

(11) EP 1 761 128 B1

(12) EUROPÄISCHE PATENTSCHRIFT

(45) Veröffentlichungstag und Bekanntmachung des Hinweises auf die Patenterteilung:
**23.02.2011 Patentblatt 2011/08**

(21) Anmeldenummer: **05751644.5**

(22) Anmeldetag: **16.06.2005**

(51) Int Cl.:
***A01N 43/56*** *(2006.01)* ***A01N 43/36*** *(2006.01)*
***A01N 37/22*** *(2006.01)* ***A01N 43/10*** *(2006.01)*
***A01N 43/78*** *(2006.01)*

(86) Internationale Anmeldenummer:
**PCT/EP2005/006483**

(87) Internationale Veröffentlichungsnummer:
**WO 2005/122770 (29.12.2005 Gazette 2005/52)**

(54) **BEIZMITTEL ZUR BEKÄMPFUNG VON PHYTOPATHOGENEN PILZEN**

PROTECTANT FOR CONTROLLING PHYTOPATHOGENIC FUNGI

AGENTS DESINFECTANTS POUR LUTTER CONTRE DES CHAMPIGNONS PHYTOPATHOGENES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **21.06.2004 DE 102004029972**

(43) Veröffentlichungstag der Anmeldung:
**14.03.2007 Patentblatt 2007/11**

(73) Patentinhaber: **Bayer CropScience AG**
**40789 Monheim (DE)**

(72) Erfinder:
- **KNEEN, Geoff**
**Chapel Hill, NC 27517 (US)**
- **SUTY-HEINZE, Anne**
**40764 Langenfeld (DE)**
- **DAHMEN, Peter**
**41470 Neuss (DE)**
- **ARAKI, Yasuo**
**Kawachi-gun,Tochigi 329-0611 (JP)**
- **SHIGYO, Takuma**
**Yokohama-shi, Kanagawa 224-0033 (JP)**
- **ELBE, Hans-Ludwig**
**42329 Wuppertal (DE)**

(56) Entgegenhaltungen:
**EP-A1- 0 737 682** **WO-A-03/010149**
**WO-A-2005/077901** **WO-A-2005/092100**
**WO-A2-2005/041653** **DE-A1- 1 567 211**
**DE-A1- 4 227 055**

- **DATABASE WPI Week 200333 Derwent Publications Ltd., London, GB; AN 2003-345929 XP002405186 & JP 2002 316902 A (SUMITOMO CHEM CO LTD) 31. Oktober 2002 (2002-10-31) -& DATABASE CA [Online] CHEMICAL ABSTRACTS SERVICE, COLUMBUS, OHIO, US; XP002405187 gefunden im STN-INTERNATIONAL Database accession no. 137:321557**

EP 1 761 128 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft die Verwendung von fungizid wirksamen Carboxamiden zur Behandlung von Saatgut gemäß Anspruch 1, sowie ein Verfahren gemäß Anspruch 4.

Es ist bereits bekannt, dass bestimmte Carboxamide fungizide Eigenschaften besitzen. So sind z.B. *N*-[2-(1,3-Dimethylbutyl)-thiophen-3 yl]-1-methyl-3-(trifluormethyl)-1H-pyrazol-4-carboxamid (EP-A 0 737 682), *N*-[2-(1,3-Dimethylbutyl)phenyl]-5-fluor-1,3-dimethyl-1*H*-pyrazol-4-carboxamid (WO 03/010149) und *N*-[2-(1,3-Dimethylbutyl)phenyl]-1-methyl-4-trifluormethyl-1H-pyrrol-carboxamid (WO 02/38542) jeweils zur Bekämpfung von phytopathogenen Pilzen durch Blattapplikation als Spritzmittel bekannt. Die Verwendung dieser Verbindungen zur Behandlung von Saatgut zum Schutz gegen den Befall von phytopathogenen Pilzen ist noch nicht bekannt geworden.

DE1567211 beschreibt die Verwendung bestimmter Oxathün-Carboxamide als Saatgutfungizide gegen *Rhizoctonia solani.*

Es wurde nun gefunden, dass Carboxamide der allgemeinen Formel (I)

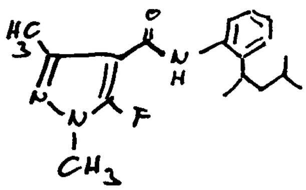

sehr gut zur Behandlung (Beizung) von Saatgut gegen Befall durch Rhizoctonia solani verwendbar sind.

Die zur Behandlung von Saatgut verwendbaren Carboxamide sind durch die Formel (I) definiert. Die Formel (I) umfasst die folgenden zur Saatgutbehandlung bevorzugt verwendbaren Carboxamide:

(I-2) *N*-[2-(1,3-Dimethylbutyl)phenyl]-5-fluor-1,3-dimethyl-1*H*-pyrazol-4-carboxamid der Formel

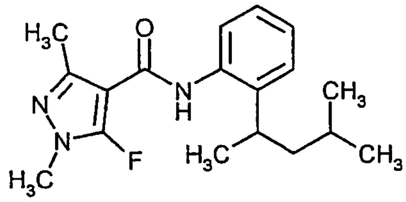

(bekannt aus WO 03/010149)

Die erfindungsgemäßen Wirkstoffe besitzen sehr gute fungizide Eigenschaften und lassen sich bei der Behandlung von Saatguts zur Bekämpfung von Rhizoctonia solani einsetzen. Die erfindungsgemäßen Wirkstoffe eignen sich bei der Behandlung von Saatgut zur Bekämpfung von Rhizoctonia solani.

Als Erreger von pilzlichen Erkrankungen, seien Rhizoctonia *solani, Rh. solani ƒ. parooketea, Rh. solani forma specialis, Rh. solani var. cedri-deodarae, Rh. solarzi var. fuchsiae, Ph. solani var. hortensis,* genannt.

Die gute Pflanzenverträglichkeit der verwendbaren Wirkstoffe in den zur Bekämpfung von Pflanzenkrankheiten notwendigen Konzentrationen erlaubt eine Behandlung des Saatguts. Die erfindungsgemäßen Wirkstoffe können somit als Beizmittel eingesetzt werden

Ein großer Teil des durch phytopathogene Pilze verursachten Schadens an Kulturpflanzen entsteht bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von phytopathogenen Pilzen, die Pflanzen nach dem Auflaufen schädigen, erfolgt in erster Linie durch die Behandlung des Bodens und der oberirdischen Pflanzenteile mit Pflanzenschutzmitteln. Aufgrund der Bedenken hinsichtlich eines möglichen Einflusses der Pflanzenschutzmittel auf die Umwelt und die Gesundheit von Menschen und Tieren gibt es Anstrengungen, die Menge der ausgebrachten Wirkstoffe zu vermindern.

Die Bekämpfung von phytopathogenen Pilzen durch die Behandlung des Saatguts von Pflanzen ist seit langem

bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch phytopathogene Pilze bestmöglich geschützt wird, ohne jedoch die Pflanze selbst durch den eingesetzten Wirkstoff zu schädigen. Insbesondere sollten Verfahren zur Behandlung von Saatgut auch die intrinsischen fungiziden Eigenschaften transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von phytopathogenen Pilzen, indem das Saatgut mit einem erfindungsgemäßen Mittel behandelt wird.

Die Erfindung bezieht sich ebenfalls auf die Verwendung der erfmdungsgemäßen Mittel zur Behandlung von Saatgut zum Schutz des Saatguts und der keimenden Pflanze vor phytopathogenen Pilzen.

Einer der Vorteile der vorliegenden Erfindung ist es, dass aufgrund der besonderen systemischen Eigenschaften der erfmdungsgemäßen Mittel die Behandlung des Saatguts mit diesen Mitteln nicht nur das Saatgut selbst, sondern auch die daraus hervorgehenden Pflanzen nach dem Auflaufen vor phytopathogenen Pilzen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Mischungen insbesondere auch bei transgenem Saatgut eingesetzt werden können.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (wie Weizen, Gerste, Roggen, Hirse und Hafer), Mais, Baumwolle, Soja, Reis, Kartoffeln, Sonnenblume, Bohne, Kaffee, Rübe (z.B. Zuckerrübe und Futterrübe), Erdnuss, Gemüse (wie Tomate, Gurke, Zwiebeln und Salat), Rasen und Zierpflanzen. Besondere Bedeutung kommt der Behandlung des Saatguts von Getreide (wie Weizen, Gerste, Roggen und Hafer), Mais und Reis zu.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt Zwischen der Ernte und der Aussaat erfolgen, Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfmdungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 200310176428 A1, WO 2002/080675 A1, WO 2002/028186 A2.

Die erfindungsgemäß verwendbaren Wirkstoffkombinationen können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe oder Wirkstoffkombinationen mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Allcylnaphthalin-Sulfonate, wie Diisopropyl- oder Düsobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylallcoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise Stoffe der Formel

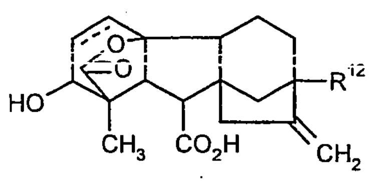

(II)

in welcher

$R^{12}$ für Wasserstoff oder Hydroxysteht und

die gestrichelte Linie andeutet, dass an der Stelle des Ringes entweder eine C-C-Einfachbindung oder eine C=C-Doppelbindung enthalten ist, in Betracht.

Als Beispiele für Gibberelline der Formel (II) seien genannt:

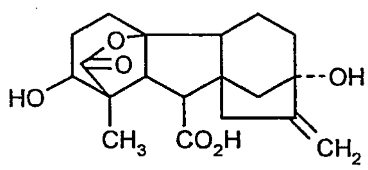

(II-1) Gibberellin A1

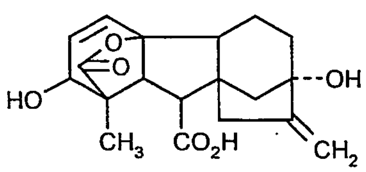

(II-2) Gibberellin A3 (= Gibberellinsäure)

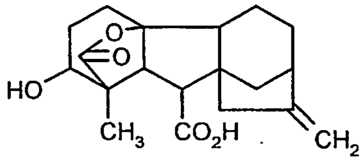

(II-3) Gibberellin A4

und

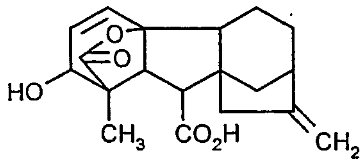

(II-4) Gibberellin A7

Besonders bevorzugt ist die Gibberellinsäure der Formel (II-2).

Die Gibberelline der Formel (II) sind bekannt (vgl. R. Wegler "Chemie der Pflanzenschutz- und Schädlingsbekämpfungsmittel", Band 2, Springer Verlag, Berlin-Heidelberg-New York, 1970, Seiten 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Wirkstoffe in den Formulierungen und nach dem Saatgut. Die Aufwandmengen an Wirkstofflcombination liegen im Allgemeinen zwischen 0,001 und 50 g pro Kilogramm Saatgut, vorzugsweise zwischen 0,01 und 15 g pro Kilogramm Saatgut.

Die gute fungizide Wirkung der erfindungsgemäß verwendbaren Wirkstoffe bei der Behandlung von Saatgut geht aus den nachfolgenden Beispielen hervor.

**Anwendungsbeispiele**

Beispiele

**Rhizoctonia solani - Test (Baumwolle) / Saatgutbechandlung**

Die Anwendung der Wirkstoffe erfolgt als Trockenbeizmittel. Sie werden zubereitet durch Abstrecken des jeweiligen Wirkstoffes mit Gesteinsmehl zu einer feinpulvrigen Mischung, die eine gleichmäßige Verteilung auf der Saatgutoberfläche gewährleistet.

Zur Beizung schüttelt man das Saatgut 3 Minuten lang mit dem Beizmittel in einer verschlossenen Glassflasche.

Das Saatgut sät man im Saatkästen mit 2 × 50 Korn in Einheitserde. Zwischen das Saatgut wird mit *Rhizoctonia solani* infizierte Perlite (5 ml/Saatkasten) gestreut, mit Lecaton abgedeckt und anschließend im Gewächshaus bei einer Temperatur von ca. 22°C und täglich 15 Stunden Licht kultiviert.

Nach 7-8 Tagen erfolgt die Auswertung der gesamten aufgelaufen und kranken Pflanzen. Die Wirkung wird nach Abbot berechnet. Dabei bedeutet 0 % ein Wirkungsgrad, der demjenigen der Kontrolle entspricht, während ein Wirkungsgrad von 100 % bedeutet, dass kein Befall beobachtet wird.

Tabelle A

| Rhizoctonia solani - Test (Baumwolle) / Saatgutbehandlung | | |
|---|---|---|
| Wirkstoff | Aufwandmenge an Wirkstoff in g/100 kg Saatgut | Wirkungsgrad in % |
| 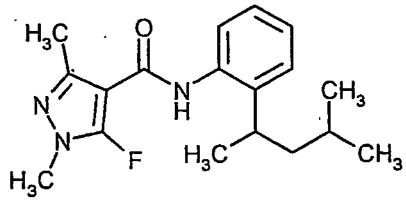 | 50 | 100 |
| 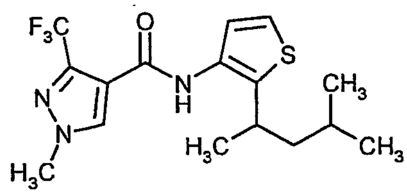 (Vergleichsbeispeil, nicht unter die Erfindung fallend) | 50 | 100 |

Beispiel B (Vergleichsbeispeil, nicht unter die Erfindung fallend)

**Pyrenophora graminea - Test (Gerste) / Saatgutbehandlung / Feldversuch**

| | |
|---|---|
| Getreideart: | Sommergerste |
| Parzellengröße: | 2 $m^2$ |
| Saatgutmenge je Parzelle: | 40g |
| Anzahl der Wiederholungen: | 3 |
| Entwicklungsstadium bei der Auswertung: | Beginn des Ährenschiebens |

Die Anwendung der Wirkstoffe erfolgt als handelsübliche Formulierung.

Zur Beizung wird das Präparat in einem Beizbecher vorgelegt und auf einen Beizschüttler gestellt. Das infizierte Saatgut wird eingefüllt und bis zur Homogenisierung ca. 2-3 min geschüttelt.

Die Aussaat im Freiland erfolgt nach praxisüblicher Bodenvorbereitung zu einem Zeitpunkt, der den Krankheitsbefall begünstigt.

Die Auswertung erfolgt zu einem Zeitpunkt, bei dem die Krankheitssymptome vollständig und gut ausgeprägt zu erkennen sind. Dabei bedeutet 0 % ein Wirkungsgrad, der demjenigen der Kontrolle entspricht, während ein Wirkungsgrad von 100 % bedeutet, dass kein Befall beobachtet wird.

Tabelle B

| Pyrenophora graminea - Test (Gerste) / Saatgutbehandlung / Feldversuch | | |
|---|---|---|
| Wirkstoff | Aufwandmenge an Wirkstoff in g/100 kg Saatgut | Wirkungsgrad in% |
| 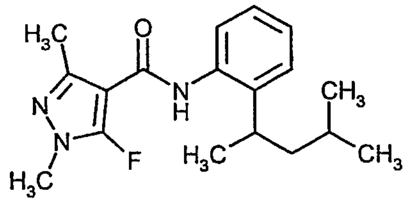 | 10 | 97 |

(fortgesetzt)

| Pyrenophora graminea - Test (Gerste) / Saatgutbehandlung / Feldversuch | | |
|---|---|---|
| Wirkstoff | Aufwandmenge an Wirkstoff in g/100 kg Saatgut | Wirkungsgrad in% |
| 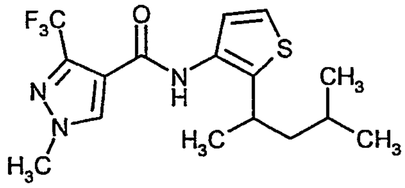 | 10 | 97 |

Der Befall lag bei 10,1 % in der Sommergersten-Sorte "Frisia".

Beispiel C

**Rhizoctonia solani - Test (Reis) / Saatgutbehandlung**

Reissaatgut (Varietät Koshihikari) wird für 10 Tage bei 15˚C in destilliertem Wasser eingeweicht, in frisches Wasser überführt und unter Belüftung für 1 Tag bei 32˚C eingeweicht. Danach wird das Saatgut für wenige Stunden bei Raumtemperatur getrocknet. Man löst 7.6 mg der Testsubstanz in 200 µl Aceton in einem Testgefäß, gibt ein Aliquot des Saatguts (entsprechend 3.8 g trockenen Saatguts) hinzu, mischt und bläst das Aceton ab. Das Saatgut wird auf Boden in einem Plastikgefäß von 7.5 cm. Durchmesser ausgesät und in einem Inkubator für 3 Tage bei 32˚C unter hoher Luftfeuchtigkeit im Dunkeln kultiviert. Anschließend werden die Sämlinge für 2 Wochen im Gewächshaus bei durchschnittlich 21˚C kultiviert. Je 5 Sämlinge werden in 5 Plastikgefäße mit einem Durchmesser von 12 cm überführt und im Gewächshaus für 5-6 Wochen bei durchschnittlich 25˚C kultiviert. Myzelien von *Rhizoctonia solani,* die auf sterilen Gerstenkörner gezogen wurden, werden am Fuß der Reispflanze ausgebracht (5 cm von der Bodenoberfläche entfernt) und in einer Glaskammer mit hoher Luftfeuchtigkeit bei durchschnittlich 27˚C inkubiert.

7 Tage nach der Inokulation wird der Grad der Infektion bewertet und der Wirkungsgrad berechnet.

Kriterien für die Bewertung des Krankheitsbefalls (Befallsrate)

| Befallsrate | Vertikale Ausdehnung der Pflanzenkrankheit (cm) |
|---|---|
| 0 | 0 |
| 0.5 | < 1.5 |
| 1 | 3-5 |
| 2 | 6-11 |
| 3 | 12 |
| 4 | 13-23. |
| 5 | > 24 |

$$\text{Wirkungsgrad}\,(\%) = \left(1 - \frac{\text{Befallsrate der behandelten Pflanze}}{\text{Befallsrate der unbehandelten Kontrolle}}\right) \times 100$$

Tabelle C

| Rhizoctonia solani - Test (Reis) / Saatgutbehandlung | | |
|---|---|---|
| Wirkstoff | Aufwandmenge an Wirkstoff in g/100 kg Saatgut | Wirkungsgrad in % |
| 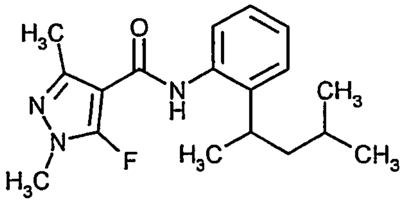 | 200 | 91 |

Beispiel D (Vergleichsbeispiel, nicht unter die Erfindung fallend)

**Ustilago nuda - Test (gerste) / Saatgutbehandlung / Feldversuch**

| | |
|---|---|
| Getreideart: | Sommergerste |
| Saatgutmenge je Parzelle: | 40g |
| Parzellengröße: | $2m^2$ |
| Anzahl der Wiederholungen: | 3 |
| Entwicklungsstadium: | Beginn bis Mitte Blüte |

Die Anwendung der Wirkstoffe erfolgt als handelsübliche Formulierung.

Zur Beizung wird das Präparat in einem Beizbecher vorgelegt und auf einen Beizschüttler gestellt: Das infizierte Saatgut wird eingefüllt und bis zur Homogenisierung ca. 2-3 min geschüttelt.

Die Aussaat im Freiland erfolgt nach praxisüblicher Bodenvorbereitung zu einem Zeitpunkt, der den Krankheitsbefall begünstigt.

Die Auswertung erfolgt zu einem Zeitpunkt, bei dem die Krankheitssymptome vollständig und gut ausgeprägt zu erkennen sind. Dabei bedeutet 0 % ein Wirkungsgrad, der demjenigen der Kontrolle entspricht, während ein Wirkungsgrad von 100 % bedeutet, dass kein Befall beobachtet wird.

Tabelle D

| Ustilago nuda - Test (Gerste) / Saatgutbehandlung / Feldversuch | | |
|---|---|---|
| Wirkstoff | Aufwandmenge an Wirkstoff in g/100 kg Saatgut | Wirkungsgrad in % |
| 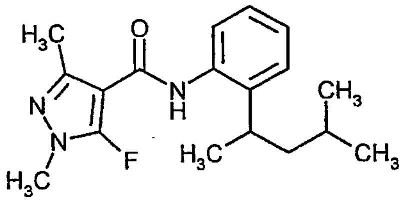 | 10 | 100 |
| 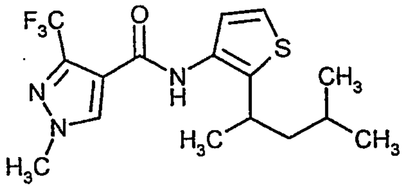 | 10 | 99 |

Der Befall lag bei 10,1 % in der Sommergersten-Sorte "Frisia".

## Patentansprüche

1. Verwendung von Carboxamiden der allgemeinen Formel (I)

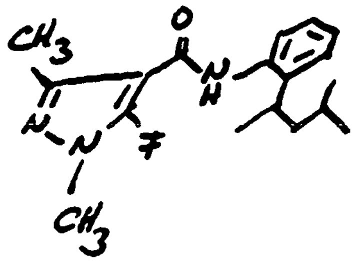

   zur Behandlung (Beizung) von Saatgut gegen Befall durch Rhizoctonia solani.

2. Verwendung gemäß Anspruch 1 zur Behandlung von Reis-Saatgut gegen den Befall von *Rhizoctonia solani.*

3. Verwendung gemäß Anspruch 1 zur Behandlung von Baumwoll-Saatgut gegen den Befall von *Rhizoctonia solani.*

4. Verfahren zum Bekämpfen von Rhizoctonia solani, **dadurch gekennzeichnet, dass** man Saatgut mit mindestens einem Carboxamid der Formel (I) gemäß Anspruch 1 behandelt.

5. Verfahren gemäß Anspruch 4 zum Bekämpfen von *Rhizoctonia solani* auf Reis-Saatgut.

6. Verfahren gemäß Anspruch 4 zum Bekämpfen von *Rhizoctonia solani* auf Baumwolle-Saatgut.

## Claims

1. Use of carboxamides of the general formula (I)

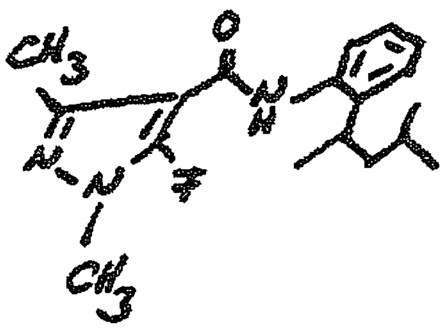

   for treating (dressing) seed against attack by Rhizoctonia solani.

2. Use according to Claim 1 for treating rice seed against attack by *Rhizoctonia solani.*

3. Use according to Claim 1 for treating cotton seed against attack by *Rhizoctonia solani.*

4. Method for controlling Rhizoctonia solani, **characterized in that** seed is treated with at least one carboxamide of the formula (I) according to Claim 1.

5. Method according to Claim 4 for controlling *Rhizoctonia solani* on rice seed.

6. Method according to Claim 4 for controlling *Rhizoctonia solani* on cotton seed.

**Revendications**

1. Utilisation de carboxamides de formule générale (I)

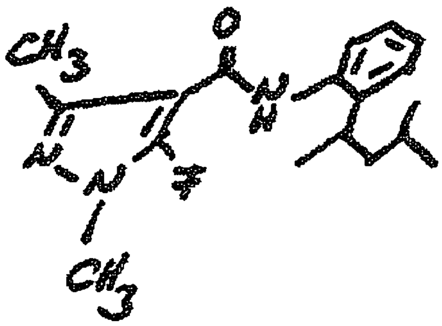

(I)

pour le traitement (la protection) de semences contre l'attaque par *Rhizoctonia solani.*

2. Utilisation selon la revendication 1 pour le traitement de semences de riz contre l'attaque par *Rhizoctonia solani.*

3. Utilisation selon la revendication 1 pour le traitement de semences de coton contre l'attaque par *Rhizoctonia solani.*

4. Procédé pour la lutte contre l'attaque par *Rhizoctonia solani,* **caractérisé en ce qu'**on traite des semences par au moins un carboxamide de formule (I) selon la revendication 1.

5. Procédé selon la revendication 4, pour la lutte contre *Rhizoctonia solani* sur des semences de riz.

6. Procédé selon la revendication 4, pour la lutte contre *Rhizoctonia solani* sur des semences de coton.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0737682 A
- WO 03010149 A
- WO 0238542 A
- DE 1567211
- US 4272417 A
- US 4245432 A
- US 4808430 A
- US 5876739 A
- US 200310176428 A1
- WO 2002080675 A1
- WO 2002028186 A2


### In der Beschreibung aufgeführte Nicht-Patentliteratur


- **R. Wegler.** Chemie der Pflanzenschutz- und Schädlingsbekämpfungsmittel. Springer Verlag, 1970, vol. 2, 401-412